# EUROPEAN PATENT APPLICATION

(11) **EP 4 640 663 A1**
(43) Date of publication of application: **29.10.2025**
(21) Application number: 24891719.7
(22) Date of filing: 08.11.2024
(51) Int. Cl.: C07C 29/82, C07C 29/04, C07C 31/10, C07C 29/80, C07C 31/12

(54) **METHOD FOR PREPARING ISOPROPYL ALCOHOL**

(30) Priority: 15.11.2023 KR 20230158482; 25.10.2024 KR 20240147789
(71) Applicant: LG CHEM, LTD., Seoul 07336 (KR)
(72) Inventor: HWANG, Sung June, Daejeon 34122 (KR); LEE, Sung Kyu, Daejeon 34122 (KR); JANG, Kyung Soo, Daejeon 34122 (KR); LEE, Moon Yong, Daejeon 34122 (KR)
(74) Representative: Goddar, Heinz J.
(86) International application number: PCT/KR2024/017592
(87) International publication number: WO 2025/105773

(57) **Abstract**

Provided is a method for preparing isopropyl alcohol including: supplying a feed including isopropyl alcohol, water, and by-products to a separation wall-type distillation column; discharging a lower discharge stream of a first region including isopropyl alcohol and by-products from a lower portion of the first region; discharging a lower discharge stream of a second region including water from a lower portion of the second region; supplying an upper discharge stream to a layer separator, refluxing an oil stream to an upper region, and refluxing an aqueous phase stream to the second region; and obtaining isopropyl alcohol from the lower discharge stream of the first region.

## Description

### [Technical Field]

### CROSS-REFERENCE TO RELATED APPLICATIONS

The present application claims the benefit of priorities to Korean Patent Application No. 10-2023-0158482, filed on November 15, 2023, and Korean Patent Application No. 10-2024-0147789, filed on October 25, 2024, the entire contents of which are incorporated herein as a part of the specification.

### Technical Field

The present invention relates to a method for preparing isopropyl alcohol, and more particularly, to a method for reducing energy usage and process costs, in the purification of isopropyl alcohol from a reaction product of an isopropyl alcohol preparation process.

### [Background Art]

Isopropyl alcohol (IPA) is acknowledged to be a solvent excellent in various industries and various uses, due to its performance to dissolve a wide range of materials, rapid vaporization, and relatively low toxicity. Isopropyl alcohol is an essential material in various manufacturing industries, health care, and direct consumer applications.

In a process of preparing isopropyl alcohol, for example, propylene and water are used as raw material components. Here, the propylene and water react to produce isopropyl alcohol. A reaction product of the process of preparing isopropyl alcohol includes water and by-products as well as isopropyl alcohol. Therefore, in order to obtain isopropyl alcohol from the reaction products, a process of purifying isopropyl alcohol is essentially involved.

For the purification of isopropyl alcohol, a plurality of distillation columns are used, and the distillation column needs much energy for vaporization of components to be separated. Accordingly, high efficiency of the purification process of isopropyl alcohol is needed, and simultaneously, an improved design is demanded in terms of economic feasibility to reduce energy usage and also reduce operation costs and equipment costs.

### [Disclosure]

### [Technical Problem]

In order to solve the problems mentioned in the Background Art, an object of the present invention is to provide a method for preparing isopropyl alcohol, which may produce high-purity isopropyl alcohol while simultaneously reducing energy usage and improving operation costs/equipment costs.

However, the object to be solved in the present application is not limited to the object mentioned above, and other objects which are not mentioned may be clearly understood by a person skilled in the art from the following descriptions.

### [Technical Solution]

In one general aspect, a method for preparing isopropyl alcohol includes: supplying a feed including isopropyl alcohol, water, and by-products to a first region of a separation wall-type distillation column which is divided into the first region, a second region, and an upper region by a separation wall and performing azeotropic distillation in the presence of an azeotropic agent; discharging a lower discharge stream of the first region including isopropyl alcohol and the by-products from a lower portion of the first region; discharging a lower discharge stream of the second region including water from a lower portion of the second region; supplying an upper discharge stream including water and the azeotropic agent, which is discharged from an upper portion of the upper region, to a layer separator, separating the stream into an aqueous phase stream including water and an oil phase stream including the azeotropic agent, refluxing the oil phase stream to the upper region, and refluxing the aqueous phase stream to the second region; and obtaining isopropyl alcohol from the lower discharge stream of the first region.

### [Advantageous Effects]

According to the method for preparing isopropyl alcohol of the present invention, in the separating of isopropyl alcohol from a feed including isopropyl alcohol, water, and a by-product, a preparation method which allows high-purity isopropyl alcohol to be obtained with fewer columns than the number of columns previously required is provided, by using a separation wall-type distillation column.

That is, according to the present invention, reboiler energy is reduced (energy saving) and reduction in installation costs and operating costs of a device may be achieved by a decrease in the number of distillation columns, by operating fewer distillation columns than the number of columns previously required.

Furthermore, separated components are discharged to first and second regions placed in the lower portion of the regions divided by the separation wall in the separation wall-type distillation column and an entire stream discharged from an upper region is cycled to the separation wall-type distillation column through a layer separator, thereby performing a conventional role of an azeotropic distillation column for separating water and isopropyl alcohol and a conventional role of an azeotropic agent recovery column for separating the azeotropic agent and water in one column, so that isopropyl alcohol having a desired purity may be separated into a lower discharge stream of the first region and pure water may be separated into a lower discharge stream of the second region, of the separation wall-type distillation column. Furthermore, an oil phase stream of the layer separator is refluxed to an upper region of the separation wall-type distillation column and simultaneously purified, thereby reducing energy which was conventionally independently used in two or more columns to reduce overall energy usage.

### [Description of Drawings]

FIG. 1 is a schematic diagram of a separation wall-type distillation column applied to a method for preparing isopropyl alcohol according to an exemplary embodiment of the present invention.
FIG. 2 is a process flow diagram of the method for preparing isopropyl alcohol according to an exemplary embodiment of the present invention.
FIG. 3 is a process flow diagram of a method for preparing isopropyl alcohol according to the comparative example.

### [Best Mode]

The terms and words used in the description and claims of the present invention are not to be construed limitedly as having general or dictionary meanings but are to be construed as having meanings and concepts meeting the technical ideas of the present invention, based on a principle that the inventors are able to appropriately define the concepts of terms in order to describe their own inventions in the best mode.

Regarding description of the drawings, similar reference numerals may be used for similar or related constituent elements.

A singular form of a noun corresponding to an item may include one or a plurality of items, unless otherwise explicitly stated in the relevant context.

In the present disclosure, each of the phrases such as "A or B", "at least one of A and B", "at least one of A or B", "A, B, or C", "at least one of A, B, and C", and "at least one of A, B, or C" may include any one of the items listed together with the corresponding phrase of the phrases or all possible combinations of them.

The term "and/or" includes a combination of a plurality of described related constituent elements or any one of a plurality of described related constituent elements.

The terms such as "1st" or "2nd", or "first" or "second" may be simply used for distinguishing a corresponding constituent element from other corresponding constituent elements, and the corresponding constituent elements are not limited in other aspects (e.g., importance or order).

In addition, the terms such as "front surface", "back surface", "upper surface", "lower surface", "side surface", "left", "right", "upper", and "lower" used in the present application are defined based on drawings, and the shape and position of each constituent element are not limited by the terms.

The terms "comprises" or "have" are intended to specify the presence of stated features, steps, operations, constituent elements, parts, or a combination thereof, but do not preclude the presence or addition of one or more other features, numerals, steps, operations, constituent elements, parts, or a combination thereof.

When it is described that a constituent element is "connected to", "combined with", "supported on", or "in contact with" other constituent elements, it includes not only the case in which the constituent elements are directly connected, combined, supported, or in contact, but also the case in which they are indirectly connected, combined, supported, or in contact through a third constituent element.

When it is described that a constituent element is positioned "on" other constituent element, it includes not only the case in which the constituent element is in contact with another constituent element, but also the case in which another constituent element is present between the two constituent elements.

The term "stream" used in the present application may refer to a fluid flow in a process, or may refer to a fluid itself flowing in a pipe. Specifically, the stream may refer to both a fluid itself flowing in a pipe connecting each device and a fluid flow. In addition, the fluid may include any one or more components of gas, liquid, and solid.

The term "upper portion" used herein refers to, unless otherwise stated, a height point of 0% to 10% downward from the top of an apparatus, and specifically, may refer to a top (tower top). In addition, the term "lower portion" refers to a height point of 90% to 100% downward from the top of an apparatus, and specifically, may refer to a bottom (tower bottom).

In addition, "pressure" mentioned herein refers to gauge pressure measured under atmospheric pressure conditions.

Meanwhile, unless otherwise particularly stated herein, operation pressure of a column refers to pressure in an upper portion of the column, and operation temperature of a column refers to a temperature in a lower portion of the column.

An exemplary embodiment of the present invention relates to a method for preparing isopropyl alcohol (IPA), and hereinafter, the method for preparing isopropyl alcohol of the present invention will be described in detail with reference to the drawings.

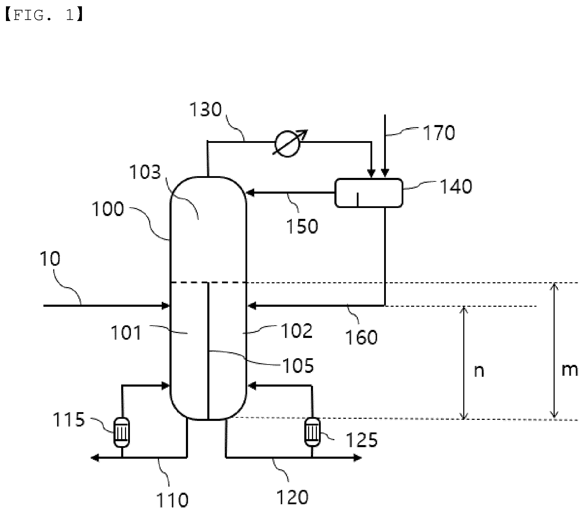

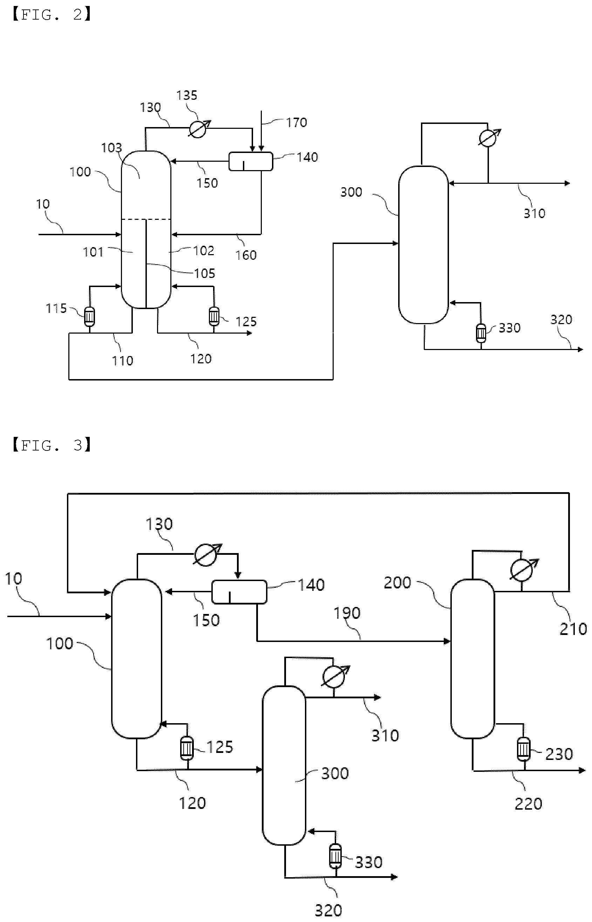

FIG. 1 is a schematic diagram of the separation wall-type distillation column according to an exemplary embodiment of the present invention, and FIG. 2 is a process flow diagram of the method for preparing isopropyl alcohol according to an exemplary embodiment of the present invention.

The method for preparing isopropyl alcohol according to the present invention includes supplying a feed 10 including isopropyl alcohol, water, and by-products to a first region 101 of a separation wall-type distillation column which is divided into the first region 101, a second region 102, and an upper region 103 by a separation wall 105 and performing azeotropic distillation in the presence of an azeotropic agent.

First, the separation wall-type distillation column 100 according to an exemplary embodiment of the present invention may include a first region 101, a second region 102, and an upper region 103 which are divided by a separation wall 105. The separation wall 105 is connected (combined) to the tower bottom of a separation wall-type distillation column 100, and is extended upward in the length direction of the separation wall-type distillation column 100 and provided. Herein, the first region 101 and the second region 102 is areas which are divided so that they face each other with the separation wall 105 therebetween, and the second region 102 is a region opposite to the first region 101. Meanwhile, the upper region 103 is a region placed above the upper end of the separation wall and is a region placed above an imaginary boundary line marked with a dotted line in FIG. 1.

Meanwhile, azeotropic distillation may be performed in the presence of an azeotropic agent in the separation wall-type distillation column 100. A part of isopropyl alcohol and a part of water included in the feed 10 may form an azeotrope. Water having a boiling point of about 100°C and isopropyl alcohol having a boiling point of about 82.3°C forms an azeotrope at an azeotropic temperature of about 81°C. Since these components may not be completely separated in the azeotrope formed as such by common distillation, isopropyl alcohol and water may be separated with high purity after removing an azeotropic relationship between isopropyl alcohol and water usually using an azeotropic agent. The azeotropic agent of the present invention which performing the function may be one or more selected from the group consisting of cyclohexane, benzene, toluene, and isopropyl acetate.

The azeotropic agent is a material which is further added separately from the feed component for azeotropic distillation, but since the azeotropic agent is an impurity in terms of isopropyl alcohol and the like, it should be separated through a separate distillation column and the like, and the separated azeotropic agent may be recycled from an economic point of view.

That is, referring to FIG. 3 related to conventional art, conventionally, in order to separate isopropyl alcohol and water from the feed 10 including isopropyl alcohol, water, and by-products, azeotropic distillation was performed in a common azeotropic column 100 which is not provided with a separation wall in the presence of an azeotropic agent, the upper discharge stream 130 including water and the azeotropic agent was phase-separated from the layer separator 140, and then an oil phase (150) including the azeotropic agent was refluxed to the azeotropic distillation column 100 again. However, since an aqueous phase still contains a large amount of the azeotropic agent in addition to water, it is introduced to an azeotropic agent recovery column 200 for the aqueous phase (190) to separate the azeotropic agent and water by distillation, the recovered azeotropic (210) agent is introduced to the azeotropic distillation column 100 again, and water (220) is discharged out of the system. Herein, in order to separate the azeotropic agent and water in the azeotropic agent recovery column 200 by distillation, a large amount of energy should be supplied through a reboiler 230 provided in the lower portion of the azeotropic agent recovery column 200.

Meanwhile, according to the conventional art, the azeotropic distillation column 100 is supplied with thermal energy required for the operation of the azeotropic distillation column 100 by a reboiler 125 provided in the lower portion. When the upper discharge stream 130 of the azeotropic distillation column 100 includes water and an azeotropic agent, the lower discharge stream of the azeotropic distillation column 100 includes isopropyl alcohol and by-products, the lower discharge stream of the azeotropic distillation column 100 is supplied to a isopropyl alcohol recovery column 300 to obtain isopropyl alcohol to the upper portion of the isopropyl alcohol recovery column 300, and the by-products (320) are separated into the lower portion of the isopropyl alcohol recovery column 300.

However, referring to FIG. 2 related to a method for preparing isopropyl alcohol according to an exemplary embodiment of the present invention, a separation wall is provided in the separation wall-type distillation column 100 which performing azeotropic distillation, and simultaneously, a reflux point of an aqueous phase and an oil phase refluxing in the layer separator 140 is optimally designed, thereby obtaining high-purity isopropyl alcohol, without being provided with a conventional azeotropic agent recovery column 200 for separating the azeotropic agent and water. Thus, thermal energy which is conventionally supplied to a reboiler (reboiler 230 in FIG. 3) for the operation of the azeotropic agent recovery column 200 may be reduced, and also cooling energy required for the operation of the condenser provided in the upper portion of the azeotropic agent recovery column 200 for the operation of the azeotropic agent recovery column 200 may be reduced. Furthermore, energy may be reduced due to the non-operation of the azeotropic agent recovery column 200, and also, even in the case of comparing the separation wall-type distillation column (distillation column 100 in FIG. 2) of the present invention with the conventional azeotropic distillation column (distillation column 100 in FIG. 3) as the distillation column, energy usage required for the operation of the distillation column may be reduced.

For this, according to an exemplary embodiment of the present invention, the first region 101 provided in the lower portion of the separation wall-type distillation column 100 may include a first reboiler 115 connected to the lower portion of the first region, and the second region 102 may include a second reboiler 125 connected to the lower portion of the second region. Herein, the lower portion refers to a height point of 90% to 100% downward from the tower top (top) of the separation wall-type distillation column 100. The first and second regions 101 and 102 may be supplied with thermal energy through a first reboiler 115 or a second reboiler 125, respectively, and the thermal energy supplied to the first reboiler 115 and the second reboiler 125 may be adjusted to adjust the operating conditions of the first and second regions 101 and 102.

According to an exemplary embodiment of the invention, the thermal energy supplied through the first reboiler 115 may be 1.5 times to 3 times, more specifically 1.8 times to 2.5 times, the thermal energy supplied through the second reboiler 125. Thus, isopropyl alcohol having a desired purity may be separated into the lower discharge stream from the first region, and pure water may be separated into the lower discharge stream from the second region of the separation wall-type distillation column.

As described above, by the supply of the thermal energy supplied to the first reboiler 115 and the second reboiler 125, a lower temperature of the first region 101 may be 80°C or higher or 82°C or higher and 100°C or lower or 95°C or lower. In addition, a temperature in the lower portion of the second region 102 may be 95°C or higher or 100°C or higher and 130°C or lower or 120°C or lower. Herein, each temperature in the lower portion is an operating temperature at a height point of 90% to 100% downward from the tower top (top) of the column 100 of the first and second regions. By controlling the temperature in the lower portion of the first and second regions, energy required for distillation of the separation wall-type distillation column 100 may be decreased, isopropyl alcohol having a desired purity may be obtained from the lower portion of the first region, and pure water may be separated from the lower portion of the second region.

Meanwhile, the lower discharge stream 110 of the first region discharged from the first region 101 of the separation wall-type distillation column 100 may include isopropyl alcohol and by-products. Herein, the by-products may include n-propyl alcohol (NPA). A part of the lower discharge stream 110 from the first region may be heat exchanged in the first reboiler 115 and then refluxed to the first region again, and the rest of the lower discharge stream 110 from the first region may be supplied to an isopropyl alcohol recovery column 300.

Meanwhile, the second region lower discharge stream 120 discharged from the second region 102 may include water. A part of the lower discharge stream 120 of the second region may be heat exchanged in the second reboiler 125 and then refluxed to the second region again, and the rest of the lower discharge stream 120 of the second region may be discharged out of the system.

As described above, a region placed above the upper end of the separation wall in the separation wall-type distillation column 100 may form an upper region 103.

The separation wall 105 is extended from the tower bottom, but the upper end of the separation wall may be placed at a height point of 10% to 45%, specifically 15% to 30%, downward from the tower top of the separation wall-type distillation column 100. Thus, the separation efficiency of the separation wall-type distillation column 100 may be maximized to obtain isopropyl alcohol having a desired purity may be obtained from the lower portion of the first region, and separate pure water from the lower portion of the second region.

An upper discharge stream 130 including water and the azeotropic agent may be discharged from the upper portion of the upper region. The upper discharge stream 130 passes through a condenser and is partially or fully condensed into a liquid phase, and the condensed stream may be introduced to a layer separator 140. The layer separator 140 is a device for separating a fluid by a density difference, and an aqueous phase including water and an oil phase including the azeotropic agent may be separated by the layer separator 140. An oil phase stream 150 including the azeotropic agent may be refluxed to the upper region 103, and an aqueous phase stream 160 including water may be refluxed to the second region 102.

That is, since the upper discharge stream 130 is separated into an oil phase and an aqueous phase by the layer separator 140 and then the separated oil phase and aqueous phase are refluxed to the separation wall-type distillation column 100 again, the mass flow rate of the upper discharge stream 130 may be the same as the sum of the mass flow rates of the aqueous phase stream 160 and the oil phase stream 150 which are refluxed to the separation wall-type distillation column 100 through the layer separator 140. That is, there is substantially no component which is, for example, supplied to other distillation columns or discharged out of the system from the upper discharge stream 130 from the separation wall-type distillation column 100. That is, the upper discharge stream 130 of the separation wall-type distillation column 100 passes through the layer separator 140 and then is fully refluxed to the separation wall-type distillation column 100.

The azeotropic agent included in the oil phase stream 150 is used in azeotropic distillation performed in the separation wall-type distillation column 100 again. Also, both the phase separated oil phase and aqueous phase are refluxed to the separation wall-type distillation column 100, but the refluxing point is optimized, thereby separating the lower discharge stream 110 of the first region and the lower discharge stream 120 of the second region with high purity. Meanwhile, the azeotropic agent 170 which is inevitably consumed as the process proceeds may be supplied to the layer separator 140 and supplemented.

That is, the stream which is discharged to other distillation columns or out of the system by the separation wall-type distillation column 100 of the present invention may have two streams of the lower discharge stream 110 of the first region including isopropyl alcohol and by-products and the lower discharge stream 120 of the second region including water.

Meanwhile, an operating pressure of the upper region 103 may be 0 kg/cm²·g or more or 0.1 kg/cm²·g or more and 3 kg/cm²·g or less or 2 kg/cm²·g or less. The operating pressure may be an operating pressure at a height at 0 to 10% downward from the tower top of the separation wall-type distillation column 100. Thus, energy required for distillation may be minimized, and simultaneously, isopropyl alcohol having a desired purity may be obtained in the lower portion of the first region and pure water may be separated in the lower portion of the second region.

A reflux point of the aqueous phase to the second region may be a point of 10% to 50%, specifically, a height point of 25% to 40% from the tower bottom of the separation wall. Specifically, referring to FIG. 1, when the height of the separation wall is m and the height of the reflux point is n from the tower bottom (m and n are expressed as the same length unit), the reflux point of the aqueous phase to the second region may be a point from the tower bottom, corresponding to a n/m ratio of the height m of the separation wall. Thus, energy required for distillation is maximized and simultaneously, pure water may be separated from the lower portion of the second region.

More specifically, when the position of the upper end of the separation wall 105 and the position of the refluxing point of the aqueous phase to the second region are set as described above, an azeotropic distillation region where isopropyl alcohol and water are separated using an azeotropic agent and a distillation region where isopropyl alcohol is purified in the separation wall-type distillation column 100 may be sufficiently secured at the same time. Furthermore, the upper region 103 in the upper separation wall shares the first and second regions, thereby reducing energy usage required in the condenser 135, and a liquid reflux stream which is branched to the first region 101 and the second region 102 in the bottom of the upper region 103 and flows downward is optimized for the first region and the second region and distributed, so that a heat amount required in the reboiler in each region may be minimized.

Meanwhile, according to an exemplary embodiment of the present invention, the lower discharge stream 110 of the first region discharged from the first region 101 may be supplied to an isopropyl alcohol recovery column 300. The isopropyl alcohol recovery column 300 is operated by a reboiler 330 placed in the lower portion, isopropyl alcohol (310) may be obtained to the upper portion of the isopropyl alcohol recovery column 300, and the by-products (320) may be separated to the lower portion of the isopropyl alcohol recovery column.

Hereinafter, the present invention will be described in more detail by the examples. However, the following examples are provided for illustrating the present invention, and it is apparent to a person skilled in the art that various modifications and alterations may be made without departing from the scope and spirit of the present invention, and the scope of the present invention is not limited thereto.

In the following example and comparative example, the method according to the present invention was simulated using a commercial process simulation program, Aspen Plus V12.1.

### Example 1

A process of purifying isopropyl alcohol was performed according to the process diagram as shown in FIG. 2.

Specifically, a feed 10 including 85.7 wt% of isopropyl alcohol, 13.57 wt% of water, and 0.73 wt% of n-propyl alcohol (NPA) as a by-product was prepared.

The feed 10 was supplied to a first region of a separation wall-type distillation column 100 and azeotropic distillation was performed in the presence of cyclohexane as an azeotropic agent. The separation wall-type distillation column 100 was a separation wall-type distillation column 100 provided with a separation wall of which the upper end is placed at point 18% downward from the tower top.

The first region 101 of the separation wall-type distillation column 100 was supplied with thermal energy from a first reboiler 115 and operated at a temperature of the lower portion of 84.3°C, and a second region 102 was supplied with thermal energy from a second reboiler 125 and operated at a temperature of the lower portion of 103°C.

An upper discharge stream 130 discharged to the upper region 103 of the separation wall-type distillation column 100 was supplied to a condenser 135 and condensed, and then the condensed stream was supplied to a layer separator 140. The stream was separated into an aqueous phase and an oil phase in the layer separator 140, and then the oil phase stream 150 including cyclohexane was refluxed to an upper region and the aqueous phase stream 160 including water was refluxed to a second region. Herein, a reflux point of the aqueous phase stream to the second region was a height point of 30% from the tower bottom of the separation wall.

Meanwhile, the lower discharge stream of the first region including isopropyl alcohol and n-propyl alcohol was supplied to an isopropyl alcohol recovery column 300, and a lower discharge stream of the second region including water was discharged out of the system.

The content of isopropyl alcohol obtained to the upper portion of the isopropyl alcohol recovery column 300 was confirmed to be 99.8 wt%. In addition, energy used in the condenser 135 (cooling energy) and energy used in the first and second reboilers 115 and 125 (thermal energy) was indicated in Table 1 based on the energy used in the comparative example.

### Comparative Example 1

A process of purifying isopropyl alcohol was performed according to the process diagram as shown in FIG. 3.

From a feed 10 identical to the feed used in Example 1, isopropyl alcohol was recovered from the upper portion of an isopropyl alcohol recovery column 300, using a common azeotropic distillation column 100 having no separation wall, an azeotropic agent recovery column 200, and an isopropyl alcohol recovery column 300. Each of the three columns had a condenser in the upper portion and reboilers 125, 230, and 330 in the lower portion.

Specifically, the feed 10 was supplied to a common azeotropic distillation column 100 to perform azeotropic distillation in the presence of cyclohexane. The upper discharge stream 130 including water and the azeotropic agent was phase-separated in a layer separator 140, and an oil phase including the azeotropic agent was refluxed to the distillation column 100 again and an aqueous phase including water was supplied to the azeotropic agent recovery column 200. The lower discharge stream of the azeotropic distillation column 100 including isopropyl alcohol and by-products was supplied to the isopropyl alcohol recovery column 300 to recover isopropyl alcohol from the upper portion.

Meanwhile, the composition and the flow rate of the lower discharge stream of the azeotropic distillation column 100 introduced to the isopropyl alcohol recovery column 300 in Comparative Example 1 were the same as those in Example 1, and the operating conditions and the used energy of the isopropyl alcohol recovery column 300 in Comparative Example 1 were also the same as those in Example 1. As a result, it was confirmed that the content of isopropyl alcohol obtained in Comparative Example 1 was 99.8 wt% as in Example 1.

At this time, energy used in each condenser of the azeotropic distillation column 100 and the azeotropic agent recovery column 200 (cooling energy) and energy used in each reboiler 125, 230 of the azeotropic distillation column 100 and the azeotropic agent recovery column 200 (thermal energy) are shown in Table 1.

**[Table 1]**

| | Comparative Example 1 | | Example 1 | |
|---|---|---|---|---|
| | Azeotropic distillation column 100 | Azeotropic agent recovery column 200 | First region | Second region |
| Energy used in condenser (condenser duty) (kW) | 78.6 | 21.4 | 42.7 | |
| Total of energy used in condenser (total condenser duty) (kW) | 100 | | 42.7 | |
| Energy used in reboiler (reboiler duty) (kW) | 78.2 | 21.8 | 28.3 | 13.7 |
| Total of energy used in reboiler (total reboiler duty) (kW) | 100 | | 42 | |
| Energy saving (%) | - | | 58 | |

For the same feed 10, Example 1 is an example in which isopropyl alcohol and by-products were separated using a separation wall-type distillation column and the isopropyl alcohol was introduced to the isopropyl alcohol recovery column to obtain isopropyl alcohol with a purity of 99.8 wt%, and Comparative Example 1 is an example in which a common azeotropic distillation column and an azeotropic agent recovery column for azeotropic agent recovery were used, but isopropyl alcohol and by-products were separated from the common distillation column and the isopropyl alcohol was introduced to the isopropyl alcohol recovery column as in Example 1 to obtain isopropyl alcohol with a purity of 99.8 wt%.

The "condenser duty" of the azeotropic distillation column and the azeotropic agent recovery column is a value distributed according to the ratio of energy actually used in the condenser of each column, when the sum of the energy usage of the condenser of each column was 100 kW. At this time, the amount of energy used in the condenser provided in the upper portion of the separation wall-type distillation column of Example 1 was 42.7 kW, and it was confirmed that the amount of energy used in the condenser was reduced.

Likewise, the "reboiler duty" of the azeotropic distillation column and the azeotropic agent recovery column of Comparative Example 1 is a value distributed according to the ratio of energy actually used in the reboiler of each column, when the sum of the energy usage of the reboiler of each column was 100 kW. At this time, the amount of energy used in each reboiler provided in the first and second regions of the separation wall-type distillation column of Example 1 was 28.3 and 13.7 kW, and the amount of energy used in the reboiler of the separation wall-type distillation column of Example 1 was a total of 42 kW, and it was confirmed that the amount of energy was reduced as compared with Comparative Example 1.

Furthermore, even considering the amount of energy required in the condenser and the reboiler which were used in the separation wall-type distillation column of Example 1 and the common azeotropic distillation column of Comparative Example 1, respectively, the separation wall-type distillation column of Example 1 was able to be operated well only with about a half the energy used in the common distillation column of Comparative Example 1.

### [Description of symbols]

10: Feed
100: Separation wall-type distillation column
200: Azeotropic agent recovery column
300: Isopropyl alcohol recovery column

## Claims

1. A method for preparing isopropyl alcohol, the method comprising:
supplying a feed including isopropyl alcohol, water, and by-products to a first region of a separation wall-type distillation column which is divided into the first region, a second region, and an upper region by a separation wall and performing azeotropic distillation in the presence of an azeotropic agent;
discharging a lower discharge stream of the first region including isopropyl alcohol and the by-products from a lower portion of the first region;
discharging a lower discharge stream of the second region including water from a lower portion of the second region;
supplying an upper discharge stream including water and the azeotropic agent, which is discharged from an upper portion of the upper region, to a layer separator, separating the stream into an aqueous phase stream including water and an oil phase stream including the azeotropic agent, refluxing the oil phase stream to the upper region, and refluxing the aqueous phase stream to the second region; and
obtaining isopropyl alcohol from the lower discharge stream of the first region.

2. The method for preparing isopropyl alcohol of claim 1,
wherein the separation wall-type distillation column includes a separation wall which is connected to a tower bottom and extended in a length direction of the column, and
is divided into the first region, the second region opposite to the first region, and the upper region placed above an upper end of the separation wall, by the separation wall.

3. The method for preparing isopropyl alcohol of claim 1, wherein an upper end of the separation wall is placed at a height point of 15 to 30% downward from a tower top of the separation wall-type distillation column.

4. The method for preparing isopropyl alcohol of claim 1, wherein the first region and the second region include first and second reboilers connected to each lower portion.

5. The method for preparing isopropyl alcohol of claim 1, wherein a temperature of the lower portion of the first region is 85°C to 100°C.

6. The method for preparing isopropyl alcohol of claim 1, wherein a temperature of the lower portion of the second region is 95°C to 115°C and is higher than the temperature of the lower portion of the first region.

7. The method for preparing isopropyl alcohol of claim 1, wherein a reflux point of the aqueous phase to the second region is a height point of 20% to 50% from a tower bottom of the separation wall.

8. The method for preparing isopropyl alcohol of claim 1, wherein the by-products include n-propyl alcohol (NPA).

9. The method for preparing isopropyl alcohol of claim 1, wherein the azeotropic agent is one or more selected from the group consisting of cyclohexane, benzene, toluene, and isopropyl acetate.

10. The method for preparing isopropyl alcohol of claim 1, wherein a mass flow rate of the upper discharge stream is equivalent to the sum of mass flow rates of the aqueous phase stream and the oil phase stream which are refluxed to the separation wall-type distillation column through the layer separator.

11. The method for preparing isopropyl alcohol of claim 1,
wherein the lower discharge stream of the first region is supplied to an isopropyl alcohol recovery column, and
isopropyl alcohol is obtained to an upper portion of the isopropyl alcohol recovery column, and the by-products are separated to a lower portion of the isopropyl alcohol recovery column.
